(19) Europäisches Patentamt
European Patent Office
Office européen des brevets

(11) Publication number: **0 130 085 B1**

# (12) EUROPEAN PATENT SPECIFICATION

(45) Date of publication of patent specification: **20.08.86**

(51) Int. Cl.[4]: **C 07 C 47/19**, C 07 C 45/75, B 01 J 31/24

(21) Application number: **84304348.0**

(22) Date of filing: **27.06.84**

(54) **Process for the production of glycol aldehyde and catalyst therefor.**

(30) Priority: **28.06.83 US 508704**

(43) Date of publication of application: **02.01.85 Bulletin 85/01**

(45) Publication of the grant of the patent: **20.08.86 Bulletin 86/34**

(84) Designated contracting states: **BE DE FR GB IT NL**

(56) References cited:
**EP-A- 0 002 908**
**EP-A- 0 061 791**
**EP-A- 0 072 051**
**EP-A- 0 096 905**
**EP-A- 0 102 341**
**GB-A- 2 114 971**
**US-A- 3 920 753**
**US-A- 4 200 765**
**US-A- 4 405 814**

(73) Proprietor: **THE HALCON SD GROUP, INC.**
**2 Park Avenue**
**New York, N.Y. 10016 (US)**

(72) Inventor: **Jacobson, Stephen E.**
**7 Ironwood Road**
**Morristown New Jersey 07960 (US)**

(74) Representative: **Cropp, John Anthony David et al**
**MATHYS & SQUIRE 10 Fleet Street**
**London, EC4Y 1AY (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 130 085 B1

Jouve, 18, rue St-Denis, 75001 Paris, France

**Description**

This invention is related to a process for the preparation of glycol aldehyde, and more particularly to the preparation of glycol aldehyde from the reaction of formaldehyde, carbon monoxide and hydrogen in a wide variety of organic solvents, and to a novel catalyst therefor.

Description of the Prior Art

Glycol aldehyde is well known as a valuable intermediate in many organic reactions, and is particularly useful as an intermediate in the production of ethylene glycol through a catalytic hydrogenation process.

The reaction of formaldehyde with carbon monoxide and hydrogen in the presence of a variety of catalysts at elevated temperatures and superatmospheric pressures is a well known reaction and yields glycol aldehyde, together with methanol, as well as lesser amounts of polyhydroxy compounds which can be subsequently separated by proper separation procedures. For example, U.S. Patent 2 451 333 describes the reaction of formaldehyde, carbon monoxide and hydrogen over a cobalt catalyst to produce ethylene glycol as an end product. U.S. Patent 3 920 753 discloses the production of glycol aldehyde by the reaction of formaldehyde with carbon monoxide and hydrogen in the presence of a cobalt catalyst under controlled reaction conditions ; however, the process produces comparatively low yields of product. Japanese patent J57-118 527 describes the production of glycol aldehyde using a ruthenium catalyst system. European Patent 002 908 describes the production of glycol aldehyde from the reaction of formaldehyde in the presence of a rhodium catalyst with carbon monoxide and hydrogen, in a tertiary amide solvent. The use of triphenyl phosphine ligand promoters is disclosed as being particularly advantageous.

U.S. Patent 4 291 179 describes a similar reaction for the production of acetaldehyde in which trifluoracetic acid is added to produce glycol aldehyde. U.S. Patent 4 356 322 describes the preparation of ethylene glycol from the reaction of synthesis gas and formaldehyde, using a rhodium or cobalt catalyst, together with ether intermediates which are hydrolyzed to ethylene glycol. European Patent Application 82/200 272.1 describes a process for the preparation of glycol aldehyde which comprises reacting formaldehyde with hydrogen and carbon monoxide in the presence of either a rhodium or cobalt-containing catalyst precursor, together with a strong protonic acid, a tertiary amide solvent and a triarylphosphine. U.S. Patent 4 200 765 describes a process of preparing glycol aldehyde which comprises reacting formaldehyde, carbon monoxide, and hydrogen in a tertiary amide solvent in the presence of a catalytic amount of rhodium in complex combination with carbon monoxide, using triphenylphosphine as the preferred promoter.

All of the known prior art processes for the production of glycol aldehyde have characteristically produced a mixture of reaction products, such as glycerine, glyceraldehyde and methyl alcohol. Unfortunately, these processes are encumbered by the need for expensive and time-consuming separation procedures, and also suffer from a consequent reduction in the amount of eventual end product produced. Such problems are particularly evident in the production of glycol aldehyde from the hydroformylation of formaldehyde, where the problems of aldol condensation reactions and of acetal formation hamper the subsequent separation of the product from the catalyst, and the formation of catalyst poisoning amines occurs, particularly when the preferred amide solvents are used. Amines also serve to catalyze the aldol condensation of glycolaldehyde with formaldehyde and other aldehydes.

Thus, it has been a major goal of the art to provide an improved process for the production of glycol aldehyde, having very high conversions and selectivities, using a variety of solvents other than amides, from the reaction of formaldehyde, carbon monoxide and hydrogen. It is further desired to provide a process wherein the desired end product can be easily and conveniently separated from the reaction mixture on a satisfactory commercial scale.

Summary of the invention

Accordingly, the invention provides for an improved process for the production of glycol aldehyde comprising contacting formaldehyde, carbon monoxide and hydrogen in a reaction zone, under suitable superatmospheric pressure and elevated temperature conditions, in the presence of a rhodium, cobalt, or ruthenium-containing catalyst, most preferably rhodium, including mixtures thereof ; the catalyst further including a carbon monoxide ligand and a phosphine ligand incorporating therein an ancillary tertiary amide group. The presence of the phosphine-amide ligand in the resultant metal carbonyl complex permits the formation of complexes having the formula, $MX_x(CO)_y[P(R_1)_2R_2(O)C—NR_3R_4]_z$, wherein M is an element selected from the group of rhodium, cobalt, and ruthenium ; X is an anion, preferably a halide, a pseudohalide, a hydride or a deprotonated strong carboxylic acid ; P is phosphorus ; $R_1$ is an aromatic or aliphatic group of 1-20 carbon atoms, preferably aromatic ; $R_2$ is an organo group containing from 0 to 20 carbon atoms of either aliphatic or aromatic nature which may include oxygen, nitrogen or sulfur atoms, which atoms may be directly bonded to the amide C(O)N carbon, or nitrogen ; $R_3$ and $R_4$ are

aliphatic or aromatic groups containing 1 to 50 carbon atoms ; the resultant compound being characterized by the absence of hydrogen on the amide nitrogen atom and the additional limitation that if $R_2$ is bonded to the amide nitrogen, then either $R_3$ or $R_4$ is bonded to the amide carbon ; x ranges from 0 to 3, y ranges from 1 to 5, and z from 1 to 4. The usage of such complexes permits a wide variety of organic polar solvents such as nitriles, ketones, ethers and the like, as well as mixtures thereof in the system, and produces higher conversions and selectivities to glycol aldehyde without being vulnerable to the prior art product recovery and purification problems.

Detailed description of the invention

The process of the present invention is accomplished by contacting formaldehyde, carbon monoxide, and hydrogen with an effective polar organic solvent, or, a mixture of polar-nonpolar organic solvents, in the presence of a rhodium, ruthenium or cobalt-containing catalyst including various mixtures thereof, the catalyst complex further including a carbon monoxide ligand and also having as a substituent a phosphine ligand incorporating an ancillary tertiary amide group, in the presence of elevated temperatures and superatmospheric pressures, that is, under general conditions suitable for hydroformylation with the particular catalyst chosen. The major product of the reaction is glycol aldehyde, with the major byproduct being methanol. The precise manner of contacting the reactants is not critical, as any of the various procedures known in the art for this type of reaction can be used so long as there is suitable efficient gas-liquid contact. Thus, for example, the process may be carried out by contacting a solution of formaldehyde together with the particular catalyst and solvent and a mixture of carbon monoxide and hydrogen at the selected conditions. Alternatively, the solution of formaldehyde may be passed over and through the catalyst in a trickle phase under a mixture of carbon monoxide and hydrogen at the selected conditions of temperature and pressure. It will, of course, be recognized that the illustrated reactants are capable of undergoing other reactions besides the primary one, and that, depending upon the particular conditions and specific catalyst and solvent chosen there will be concomitant production of other products in variable amounts, particularly methanol. However, the conditions in the instant process are most preferably regulated so as to give high selectivity to the desired glycol aldehyde.

A suitable source of formaldehyde can be any one of those known in the art, including paraformaldehyde, methylal, formalin solutions, polyoxymethylenes and the like. Of these, paraformaldehyde is preferred and maximum yields have been obtained from its use.

While carbon monoxide and hydrogen react in a stoichiometric one-to-one ratio, it is not necessary to have them present in such a ratio to undertake the reaction. As indicated above, the reactant should be employed at a high enough pressure so as to provide a desirable reaction rate. Carbon monoxide also stabilizes rhodium and the other metals to reduction by formaldehyde to the zero valent state. The carbon monoxide and hydrogen reactants may conveniently be supplied in about a one-to-one ratio, on a mole basis, such as can be obtained from synthesis gas and the like ; however, they can also be present in widely varying and variable ranges, such as having mole ratios from about 5 to 95 to 95 to 5. Excellent yields can be obtained when operating at carbon monoxide to hydrogen partial pressure ratios as high as 10 to 1. Large excesses of hydrogen have a tendency to favor the production of unwanted methanol, while it is often desirable to have at least an equimolar amount of carbon monoxide present, particularly if temperature or other reaction conditions are such so as to present a need to suppress methanol production.

A major problem with this reaction as practised by the prior art has been that glycol aldehyde tends to form acetals, a reaction typical of aldehydes. Since there is a primary alcohol group present in the molecule, this compound can easily form semi-acetals and acetals with itself in the form of, for example, linear and cyclic-acetals such as are represented by the following structures.

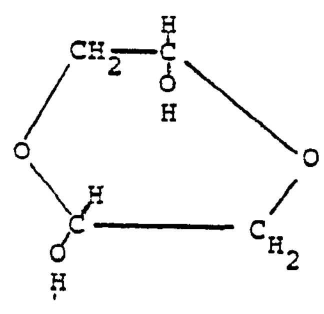 , 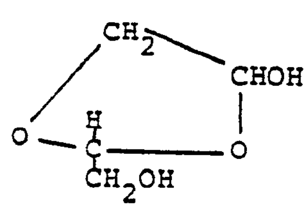 , 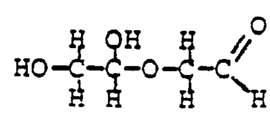 .

In addition, glycol aldehyde also forms acetals and semi-acetals with methanol, and if present, ethylene glycol. Acetals are resistant to hydrogenation, as well as possessing high boiling points, and therefore can present a difficult process impediment to ethylene glycol formation. Also, the glycol aldehyde product can react with either formaldehyde or with itself through the mechanism of aldol condensation. Finally, the reactions can be inhibited by the product glycol aldehyde which can form

bidentate ligands with the coordination metal, i. e., rhodium, and thereby displace a monodentate phosphine ligand.

Surprisingly, it has been discovered that these and other related problems can be substantially eliminated through the use of a novel coordination metal compound, e. g., by the use of a coordination metal, most preferably rhodium, phosphine ligand having incorporated therein an ancillary tertiary amide group, which forms a catalyst which can hydroformylate formaldehyde to glycol aldehyde in very high selectivities, and, surprisingly, in the absence of an amide solvent and which is also operative in a variety of polar organic solvents, as well as nonpolar solvents, and various mixtures thereof.

The coordination metal compound, suitable for use in the reaction is selected from either rhodium, cobalt or ruthenium, as well as various mixtures thereof, and most preferably a rhodium compound, complex, or salt, as well as certain mixtures. The metal compound may be deposited or affixed to a solid support such as a molecular sieve, zeolite, activated carbon, alumina, silica, an ion exchange resin, an organic polymeric resin, or as an insoluble rhodium oxide, but most preferably is used as a homogeneous complex in solution.

A particular broad class of metal complexes or precursors which have been found to be generally suitable can be represented by the equation :

$$MX_x(CO)_y[P(R_1)_2R_2(O)C{-}NR_3R_4]_z$$

wherein M is an element selected from the group of rhodium, cobalt and ruthenium, including mixtures thereof, X is an anion which can be a halide, a pseudohalide, a hydride or a deprotonated strong carboxylic acid, P is phosphorus, $R_1$ is an aliphatic, aromatic or a mixture thereof, group of 1-20 carbon atoms attached directly to the phosphorus atom, $R_2$ is an organo group containing from 0 to 20 carbon atoms of either aliphatic or aromatic nature, and may include such atoms as oxygen, nitrogen, sulfur and the like, which atoms may also be directly attached to the amide carbon or nitrogen ; $R_3$ and $R_4$ are organic, aliphatic or aromatic moieties of 1 to 50 carbon atoms, the resultant amide moiety being characterized by the absence of hydrogen on the amide nitrogen and the additional limitation that if $R_2$ is bonded to the amide nitrogen, then either $R_3$ or $R_4$ is bonded to the amide carbon ; x ranges from 0 to 3, y ranges from 1 to 5, and z from 1 to 4.

The anion element X in the aforementioned complex can be a hydride, a halide such as chloride, bromide, iodide or fluoride, as well as pseudohalides which exhibit halide like properties in forming coordinate compounds and the like, including substituents such as $NCS^-$, $NCO^-$, $CN^-$, $NCSe^-$, $N_3^-$,

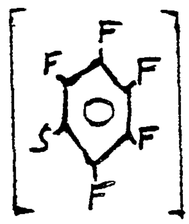 − 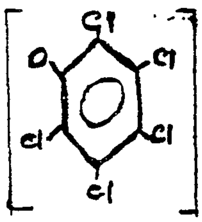 −,

and the like, or a deprotonated strong carboxyclic or sulfonic acid substituent such as trifluoroacetate, trifluoromethanesulfonate, methanesulfonate, toluenesulfonate and the like. The preferred anion containing moieties are chloride, trifluoroacetate and hydride. Alternatively, an anionic rhodium species can be generated in which no anionic element X is present.

The $R_1$ group, in the broadest embodiment, represents either an alkyl, an aryl group or a mixture thereof, the alkyl group containing from 1-20 carbon atoms, with the suitable aryl moieties being phenyl and substituted phenyls, other polyaromatics and substituted polyaromatics and the like. When cobalt is the coordinating metal ligand of choice, it is preferred to use alkyl substituted phosphine, whereas when rhodium or ruthenium is the chosen metal it is preferred to have $R_1$ be phenyl or another aryl moiety.

The $R_2$ group, in the broadest embodiment, represents an organo group containing from 0 to 20 carbon atoms, preferably from 1 to 2 carbon atoms, and is preferably free from acetylenic unsaturation. $R_2$ can be saturated aliphatic, alkenyl, or aromatic and can be either a hydrocarbyl group containing only carbon and hydrogen, or, a substituted hydrocarbyl group containing in addition to atoms of carbon and hydrogen additional atoms such as nitrogen, oxygen, sulfur and the halogens, which additional atoms can also be present in various groups such as alkoxy, aryloxy, carboalkoxy, alkanoyloxy, halo, trihalomethyl, cyano, sulfonylalkyl and the like groups which contain no active hydrogen atoms. Most preferably, $R_2$ is comprised of aliphatic groups containing only carbon and hydrogen.

Illustrative of suitable $R_1$ and $R_2$ moieties are hydrocarbon alkyls such as methyl, ethyl, propyl, isobutyl, cyclohexyl, and cyclopentyl ; hydrocarbon alkenyl moieties such as butenyl, hexenyl, cyclohexenyl ; alkyl or alkenyl moieties having aromatic substituents such as benzyl, phenylcyclohexyl and phenylbutenyl ; and substituted-hydrocarbyl moieties such as 4-bromohexyl, 4-carbethoxybutyl, 3-cyanopropyl, chlorocyclohexyl and acetoxybutenyl. Aromatic moieties are exemplified by hydrocarbyl aromatic groups such as phenyl, tolyl, xylyl, p-ethylphenyl, and substituted hydrocarbyl aromatic groups

such as p-methoxyphenyl, m-chlorophenyl, m-trifluoromethylphenyl, p-propoxyphenyl, p-cyanophenyl, o-acetoxyphenyl and m-methyl-sulfonylphenyl.

$R_3$ and $R_4$ represent organo groups of 1 to 50 carbon atoms preferably from 1 to 6 carbons, and can be either aromatic, or preferably aliphatic in nature. Suitable groups are the saturated aliphatics of both a cyclic or a linear makeup, aromatics, preferably mononuclear aromatics, and the like. It is most preferred that $R_3$ and $R_4$ be comprised of only carbon and hydrogen atoms. It is also most preferred that the amide moiety in the resultant phosphine ligand be characterized by the absence of hydrogen on the amide nitrogen atom ; e. g. tertiary amide groups are most preferred. In the case where $R_2$ is bonded to the nitrogen, instead of the amide carbon, then either $R_3$ or $R_4$ is bonded to the amide carbon. A second organophosphine group can also be incorporated into one or both of the $R_3$ or $R_4$ groups.

A representative phosphine-amide ligand can be synthesized through a modification of the method disclosed by Meek et al, (J. Chem. Soc. (Dalton), 1 011, 1975), in which a phosphorus hydrogen bond cleanly adds to the carbon-carbon double bonds of vinyl derivatives in the presence of free radicals such as 2,2′-azobis (2-methyl-propionitrile)(AIBN), e. g. :

$$PPh_2H + CH_2 = CH{-}C(O)N(CH_3)_2 \xrightarrow[100\ ^\circ C]{AIBN} PPh_2CH_2CH_2C(O)N{-}(CH_3)_2.$$

In this case the resulting phosphine exists as a white solid at room temperature. Alternatively, a vinyl amide can be reacted with the secondary phosphine as above. The phosphine-amide can also be synthesized by other routes, such as by photochemistry or by amination of a phosphine ester.

The catalyst can be prepared through a variety of techniques, e. g., the complex containing carbon monoxide can either be preformed or formed *in situ* by the reaction with the coordination metal. Several convenient methods for *in situ* preparation are to contact a metal complex precursor with the phosphine-amide and an acid such as trifluoroacetic acid ; to contact a metal carbonyl halide, e. g. $[RhCl(CO)_2]_2$ with the phosphine-amide ; to contact a preformed metal coordination complex such as $Rh(Cl(CO)(PPh_3)_2$ with the desired phosphine-amide ; or to contact the metal carbonyl halide, e. g. $[RhCl(CO)_2]_2$ with a catalytic amount of base and the phosphine-amide.

Although it is not wished to be bound by theory, the reason for the selectivity of the formaldehyde hydroformylation to glycol aldehyde when using the coordination metal phosphine-amide in nonamide solvents is most likely to be found in the size and resulting stability of the formed chelate ring. Depending on the particular coordination metal used, and, to a lesser extent, the particular phosphine-amide ligand employed, a variety of desired bidentate complexes can be created, which complexes apparently bond with the coordination metal at the phosphorus and the amide group oxygen, thus creating a variety of bidentate complexes. For example, one of the preferred phosphine-amide ligands, N,N-dimethyl-3-diphenylphosphinopropionamide, $PPh_2CH_2CH_2C(O)N(CH_3)_2$ is believed to form a 6 membered ring with rhodium and is surprisingly active, as well as selective to glycol aldehyde.

A number of suitable phosphine-amide ligands are exemplified below :

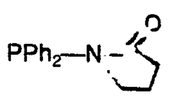 ;

$PPh_2OCH_2C(O)N(CH_3)_2$;

$PPh_2N(CH_3)CH_2C(O)N(CH_3)_2$;

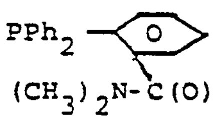

$PPh_2CH_2CH_2N(CH_3)C(O)CH_3$;

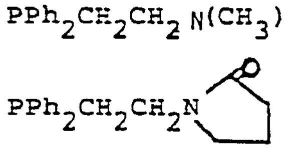 (5)

$P(CH_2CH_2CH_2CH_3)_2[CH_2CH_2C(O)N(CH_3)_2]$'

$PPh_2CH_2CH_2C(O)N(CH_3)(CH_2CH_2CH_2CH_2CH_2CH_2CH_2CH_3)$;

$PPh_2CH_2C(O)N(CH_3)_2$; $PPh_2CH_2N(CH_3)C(O)CH_3$

$PPh_2CH_2CH_2C(O)N(CH_3)CH_2PPh_2$

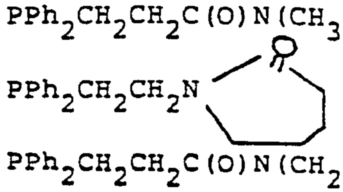

$PPh_2CH_2CH_2C(O)N(CH_2Ph)(CH_2CH_3)$

$PPh_2CH_2CH_2C(O)N(Ph)(CH_2CH_2CH_2CH_3)$

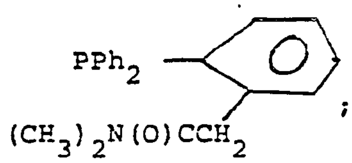

;

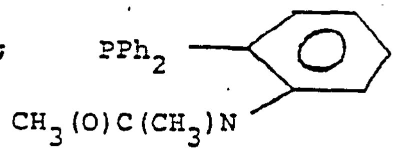

$PPh_2CH_2C(O)N(Ph)_2$

The usage of this novel class of complexes as catalysts has surprisingly permitted the use of a great many more different solvents than have heretofore been found effective in such reactions. Although certain nonpolar solvents, as well as mixtures, have proven to be operable, the use of polar solvents, and particularly organic polar solvents has been found to be generally preferred. Particularly preferred as solvents are nitriles, such as acetonitrile, benzonitrile, propionitrile and the like ; cyclic ethers such as tetrahydrofuran, dioxane and tetrahydropyran ; ethers such as diethyl ether, 1,2-dimethoxybenzene, alkyl ethers of alkylene glycols and polyalkylene glycols, e. g., methyl ethers of ethylene glycol, propylene glycol and di-, tri-, and tetraethylene glycols ; alkyl sulfones and sulfoxides such as sulfolane and dimethylsulfoxide ; ketones such as acetone, methyl isobutyl ketone, and cyclohexanone ; esters such as ethyl acetate, ethyl propionate and methyl laurate ; lactones of organic carboxylic acids such as butyrolactone and valerolactone, organic acids such as acetic acid, propionic acid and caproic acid, and alkanols, such as methanol, ethanol, propanol, 2-ethylhexanol and the like ; as well as mixtures thereof. Nonpolar organic solvents such as benzene, toluene and the like are also operable if used in mixtures with polar organics. The selected solvent should preferably be liquid under the reaction conditions.

A major factor to be considered in choosing a solvent, particularly for an industrial scale operation, is its suitability for subsequent product separation procedures. The use of nonamide solvents is particularly helpful in product separation procedures since their use disposes of having to separate the formed glycol aldehyde from the amide solvents in which aldol condensation and other competing side reactions occur. Other serious flaws of amide solvents are their tendency to form amines during the reaction process, and their high boiling points which can result in solvent decomposition during distillation. Amines, particularly tertiary amines, have been found to act as a poison in these reactions when present in suitable concentrations, as well as catalyzing aldol condensation ; thus their presence is particularly undesirable. The only way in which amides can be separated from amine contamination is by distillation. Consequently, in any process system where maximum yields of glycol aldehyde are desired, it is a basic goal in the operation to avoid the presence of basic amines in any significant amount.

An additional improvement resulting from the catalyst complexes described herein is that in solution the phosphine-amides act as bidentate ligands which are more resistant to displacement by glycol aldehyde than the prior art monodentate phosphines. This means that, in sharp contrast to prior art systems, the glycol aldehyde product does not have to be removed from the reaction media shortly after its formation so as to ensure the stability of the catalyst. Such properties are extremely helpful in optimizing product yield and facilitating product and catalyst separation. The reaction can also be carried out at higher concentrations of formaldehyde without any harmful side reactions.

As in other processes of this kind, the reaction can be conducted in either a batch, semi-continuous or continuous mode of operation. It is naturally desirable to construct the reactor from materials which can withstand the operating temperatures and pressures required, while keeping the internal surfaces of the reactor substantially inert. Standard equipment known to permit control of the reaction, such as heat exchangers and the like, may be used. The reactor should be provided with an adequate means for agitating the reaction mixture ; such mixing can be induced by vibration, shaking, stirring, oscillation, and similar type methods. Also, recovered catalyst, solvent and unreacted starting materials are preferably recycled.

The reaction resulting in the production of glycol aldehyde, together with lesser amounts of methanol, is usually substantially complete within a pariod of about 2-3 hours, although reaction time is not a critical parameter of the process, and longer or shorter times can be effectively employed. Substantially higher concentrations of reactants than previously utilized are preferred, and the reaction also yields higher conversions of reactants than the prior art.

The amount of catalyst employed in the hydroformylation reaction process has not been found to be

critical and can vary considerably. At least a catalytically effective amount of catalyst should be present, and preferably a sufficient amount of catalyst which is effective to provide a reasonable reaction rate. As little as $10^{-5}$ moles of catalyst per liter of reaction medium can suffice, while amounts in excess of $10^{-1}$ moles do not appear to materially affect the rate of reaction. For most purposes, an effective preferred amount of catalyst falls in the range of from about $10^{-2}$ to about $10^{-3}$ moles per liter.

The glycol aldehyde can be extracted with water, and followed by hydrogenation to ethylene glycol, using heterogeneous catalysts known in the art. However, other extraction techniques and product utilization plans may, of course, be used. Alternatively, the glycol aldehyde can be hydrogenated to ethylene glycol using the metal phosphineamide catalyst system in the organic solvent used for the hydroformylation.

The precise reaction conditions chosen are not particularly critical in that a wide range of elevated temperatures and superatmospheric pressures are operable. Using current available apparatus, the preferred temperature range should be at least about 50 °C and can range up to about 150 °C and even higher, although no substantial benefits are realized at this temperature level. Most preferably, the operating temperatures will range from about 90 °C to about 130 °C. The superatmospheric pressures should be at least about 70 atmospheres and can range, in theory, to almost any pressure attainable with available production apparatus. Most preferably, operating pressures should fall in the range of about 140 to about 280 atmospheres, particularly when operating in the preferred aforementioned temperature range.

The following examples are provided to illustrate the invention in accordance with the principles of this invention but are not construed as limiting the invention in any way except as indicated by the appended claims.

## Examples

The phosphine-amides were prepared by the following general methods :

Preparation of N,N-dimethyl-3-diphenylphosphinopropionamide [$PPh_2CH_2CH_2C(O)N(CH_3)_2$]

A 100 ml 3-necked round bottomed flash was preheated to 100 °C under a nitrogen atmosphere and 7.14 g diphenylphosphine (38.4 mmole) from Alfa, 0.15 g 2,2′-azobis (2-methylpropionitrile) from Aldrich, and 3.78 g dimethylacrylamide (38.2 mmole) from Polysciences were added in the stated sequence. The reaction mixture was maintained at 100 °C under a nitrogen atmosphere for 45 minutes and then evacuated (1 mm) for 24 hours at 100 °C. The resulting oil was then cooled in the refrigerator for several hours until it solidified to a white solid. It was then washed with a 9 : 1 pentane : toluene mixture so as to free the excess diphenylphosphine.

The infrared spectra disclosed a strong $\nu$(CO) band at 1 650 $cm^{-1}$. The $^1H$ NMR showed a multiplet at 2.2-2.5γ with a relative area of 4 assigned to the $CH_2CH_2C(O)$ group ; a doublet at 2.83γ (3 Hz) of relative area 6 assigned to the $N(CH_3)_2$ group ; and a multiplet at 7.1-8.3γ of relative area of 10 assigned to the $P(C_6H_5)_2$ group.

The elemental analysis was the following : calculated (found) ; C, 71.58 (70.95) ; H, 7.02 (7.03) ; N, 4.91 (4.05) ; P, 10.87 (10.75). Product Yield was 71 %.

Preparation of N-methyl-N-(2-diphenylphosphinoethyl) acetamide [$PPh_2CH_2CH_2N(CH_3)C(O)CH_3$]

A 100 ml 3-necked round bottomed flash was heated to 100 °C under a nitrogen atmosphere and 7.14 g diphenylphosphine (38.4 mmole), 0.15 g 2,2′-azobis (2-methylpropionitrile), and 3.77 g N-vinyl-N-methyl acetamide (38.1 mmole) from Polysciences were added in the stated sequence. The reaction was maintained at 100 °C for 45 minutes under a nitrogen atmosphere and then evacuated (1 mm) for 24 hours at 100 °C. A viscuous oil resulted whose infrared spectrum displayed a strong $\nu$(CO) band at 1 645 $cm^{-1}$ and a small $\nu$(P-H) band at 2 300 $cm^{-1}$ due to a small amount of diphenylphosphine impurity. The elemental analysis was the following : calculated (found) ; C, 71.58 (69.89) ; H, 7.02 (7.15) ; N, 4.91 (4.57) ; P, 10.87 (10.97). The yield was 94 %.

Preparation of N-(-2-diphenylphosphinoethyl)-2-pyrrolidinone ($PPh_2CH_2CH_2N$)

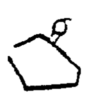

The cited phosphine-amide was prepared in a similar manner to the earlier two examples by using 14.28 g diphenylphosphine (76.8 mmole), 0.30 g 2,2′-azobis (2-methylpropionitrile) and 8.52 g N-vinyl-2-pyrrolidinone (76.8 mmole) from Aldrich. The reactants added in the stated sequence were stirred at 100 °C for 0.5 hours under a nitrogen atmosphere followed by evacuation (1 mm) for 24 jours. The yield was 96 %.

The infrared spectrum disclosed a strong, broad υ(CO) band at 1 680 $cm^{-1}$ and a small absorption at 2 310 $cm^{-1}$ due to a small amount of υ(P-H) impurity. The elemental analysis was the following : calculated (found) ; C, 72.70 (72.68) ; H, 6.79 (6.73) ; N, 4.70 (3.49) ; P, 10.42 (11.60). The $^1$HNMR showed a multiplet at 1.6-2.6γ with a relative area of 6 assigned at $—CH_2CH_2C(O)—$ and $P—CH_2—$ groups, a multiplet at 3.2-3.8γ with a relative area of 4 assigned to the two $CH_2—N—$groups and a multiplet at 7.2-8.0γ with a relative area of 11 assigned to the $P(C_6H_5)_2$ group. A small amount of diphenylphosphine impurity accounts for this slightly greater area for the phenyl region. The disappearance of the multiplet signals of the vinyl protons at 6.9-7.4γ and 4.3-4.7γ confirms the complete disappearance of N-vinylpyrrolidinone.

The following examples illustrate a direct comparison between the phosphine-amides of this invention with triphenylphosphine and in one case ethyldiphenylphosphine (another alkyldiphenylphosphine) in various solvents.

## Example 1

A 300 cc stainless steel autoclave equipped with a stirrer, thermocouple, and cooling coil was charged with 100 g acetonitrile, 3.0 g of 95 % paraformaldehyde (95 mmole of equivalent formaldehyde), 0.081 g chlorodicarbonylrhodium (I) dimer (0.208 mmole) and 0.364 g N,N-dimethyl-3-diphenylphosphinopropionamide (1.28 mmole), which was sparged with dry nitrogen. The autoclave was sealed and the air was further removed by flushing the autoclave three times with a 1 : 1 mixture of carbon monoxide : hydrogen at 689 KPa (100 psi). The autoclave was then charged with 1 500 psi of the carbon monoxide : hydrogen mixture. The autoclave and its liquid contents were next heated to 130 °C and the final pressure registered 13.1 MPa (1 900 psi). After three hours, the autoclave was cooled to room temperature and the gas was vented. Gas chromatography and high pressure liquid chromatography of the resulting liquid phase disclosed 37 mmole of formaldehyde remaining (61 % conversion) with 35 mmole glycol aldehyde (60 % selectivity) and 4.1 mmole of methanol (7.1 % selectivity). The selectivity is defined as mmoles of product divided by mmoles of reacted formaldehyde times 100 in all examples.

## Comparative Example 1

An equimolar concentration of triphenylphosphine was substituted for N,N-dimethyl-3-diphenylphosphinopropionamide, under the identical conditions of Example 1. Analysis of the liquid phase showed 53 mmole of formaldehyde remaining (44 % conversion), with 7.6 mmole of glycol aldehyde and 0.8 mmole of ethylene glycol (20 % selectivity) and 8.0 mmole of methanol (19 % selectivity).

## Second Comparative Example 1

An equimolar concentration of ethyldiphenylphosphine was substituted for N,N-dimethyl-3-diphenylphosphinopropionamide, under the identical conditions of Example 1. Analysis of the liquid phase showed 5.5 mmole formaldehyde remaining (94 % conversion) with 0.6 mmole glycol aldehyde (0.7 % selectivity) and 1.6 mmole methanol (1.8 % selectivity).

## Example 2

A 125 cc stainless steel Parr bomb equipped with a glass liner and a magnetic stirrer was charged with 25.0 g acetonitrile, 0.75 g paraformaldehyde (23.8 mmole), 0.020 g chlorodicarbonylrhodium (I) dimer (0.058 mmole) and 0.090 g N,N-dimethyl-3-diphenylphosphinopropionamide (0.348 mmole) which had been sparged with nitrogen. The bomb was then charged with 10.3 MPa (1 500 psi) of a 1 : 1 carbon monoxide : hydrogen mixture, heated to 130 °C for 3.0 hours, and then cooled to room temperature and vented. Analysis of the liquid contents indicated 9.3 mmole formaldehyde remaining (61 % conversion), 11.9 mmole glycol aldehyde (82 % selectivity), and 0.2 mmole methanol (1.4 % selectivity).

## Comparative Example 2

The bomb used in Example 2 was charged and reacted under identical conditions to those of Example 2 except the catalyst was chlorocarbonylbis(triphenylphosphine) rhodium (I) (0.080 g, 0.116 mmole) in the absence of N,N-dimethyl-3-diphenylphosphinopropionamide as co-catalyst. Analysis of the liquid contents indicated 3.8 mmole formaldehyde remaining (84 % conversion), 0.5 mmole glycol aldehyde (3 % selectivity), and 0.4 mmole methanol (2 % selectivity).

## Example 3

The Parr bomb used in Example 2 was charged with 25.0 g acetonitrile, 0.75 g paraformaldehyde (23.8 mmole), 0.080 g chlorocarbonylbis(triphenylphosphine) rhodium (I) (0.116 mmole), and 0.060 g N,N-dimethyl-3-diphenylphosphinopropionamide (0.232 mmole) which had been sparged with nitrogen. The bomb was then charged with 10.3 MPa (1 500 psi) of a 1 : 1 carbon monoxide : hydrogen mixture at room temperature and subsequently heated to 130 °C for 3.0 hours. After cooling and gas venting, the analysis indicated 7.6 mmoles of formaldehyde remaining (68 % conversion), 11.1 mmoles glycol aldehyde (69 % selectivity), and 5.0 mmoles methanol (31 % selectivity).

Example 4

The Parr bomb used in Example 2 was run under identical conditions to Example 2 except that an equimolar concentration of N-methyl-N-(2-diphenylphosphinoethyl) acetamide was substituted for N,N-dimethyl-3-diphenylphosphinopropionamide. Analysis of the liquid contents indicated 13.4 mmole formaldehyde remaining (44 % conversion), 5.3 mmole glycol aldehyde (51 % selectivity), and 2.1 mmole methanol (20 % selectivity).

Example 5

The autoclave used in Example 1 was charged with 9.0 g paraformaldehyde (285 mmole), 65.0 g acetonitrile, 30.0 g toluene, 5.0 g diethyl ether, 0.301 g N,N-dimethyl-3-diphenylphosphinopropionamide (1.06 mmole), and 0.069 g chlorodicarbonylrhodium (I) dimer (0.178 mmole). The autoclave was charged with 12.1 MPa (1 750 psi) carbon monoxide and 3.1 MPa (450 psi) hydrogen at room temperature. A 2 liter autoclave reservoir was also charged with this same carbon monoxide : hydrogen ratio and heated to 250 °C to a total pressure of 24.8 MPa (3 600 psi). The reactor autoclave was heated to 130 °C for 3.0 hours to a total pressure of 24.8 MPa (3 600 psi). When the gas uptake lowered the gas pressure to 22.1 MPa (3 200 psi), gas was transferred from the reservoir autoclave to the reactor autoclave during the run. Upon completion, the autoclave was cooled to room temperature and slowly vented. Analysis of the liquid products revealed 136 mmole formaldehyde remaining (52 % conversion), 120 mmole glycol aldehyde (81 % selectivity) and 12.9 mmole methanol (8.7 % selectivity).

Comparative Example 5

A comparative example, using an equimolar quantity of triphenylphosphine instead of N,N-dimethyl-3-diphenylphosphinopropionamide under identical conditions, resulted in a liquid phase containing 174 mmole formaldehyde remaining (39 % conversion), with 27.3 mmole glycol aldehyde (25 % selectivity) and 25.2 mmole methanol (23 % selectivity).

Example 6

The autoclave in Example 5 was charged with 9.0 g paraformaldehyde (285 mmole), 65.0 g acetonitrile, 30.0 g toluene, 5.0 g diethyl ether, 0.315 g N(-2-diphenylphosphino-ethyl)-2-pyrrolidinone (1.06 mmole), and 0.069 g chlorodicarbonylrhodium (I) dimer (0.178 mmole). The autoclave was then charged with 12.1 MPa (1 750 psi) carbon and 3.1 MPa (450 psi) hydrogen at room temperature. The 2 liter autoclave reservoir was also used, as in Example 5. The reactor autoclave was heated to 130 degrees C for 3.0 hours. Analysis of the liquid products revealed 137 mmole formaldehyde remaining (52 % conversion), 143 mmole glycol aldehyde (97 % selectivity) and 3.6 mmole methanol (2 % selectivity).

Example 7

The autoclave of Example 5 was charged with 100 g tetraglyme, 9.0 g paraformaldehyde (285 mmole), 0.069 g chlorodicarbonylrhodium (I) dimer (0.177 mole), and 0.301 g N,N-dimethyl-3-diphenylphosphinopropionamide (1.06 mmole). The gas reservoir of Example 5 was again used in the same manner. The autoclave was charged with 12.1 MPa (1 750 psi) carbon monoxide and 3.1 MPa (450 psi) hydrogen at room temperature and then heated to 130 °C for 3.0 hours to a total pressure of 24.9 MPa (3 600 psi). The autoclave was cooled, vented, and the liquid contents analyzed as 104 mmole formaldehyde remaining (64 % conversion), 111 mmole glycol aldehyde and 1.2 mmole ethylene glycol (62 % selectivity), and 14.6 mmole methanol (8.1 % selectivity).

Comparative Example 7

A comparative example using an equimolar concentration of triphenylphosphine instead of N,N-dimethyl-3-diphenylphosphinopropionamide under identical conditions resulted in a liquid phase containing 75 mmole formaldehyde remaining (74 % conversion), 7.6 mmole glycol aldehyde (3.6 % selectivity), and 5.1 mmole methanol (2.4 % selectivity).

Example 8

The autoclave and reservoir of Example 5 were again run under identical conditions and the charge

was identical except that 100 g acetone was used as the solvent. Analysis of the liquid contents indicated 106 mmole of formaldehyde remaining (63 % conversion), with 145 mmole glycol aldehyde (81 % selectivity) and 10.2 mmole methanol (5.7 % selectivity).

Comparative Example 8

A comparative example using an equimolar concentration of triphenylphosphine instead of N,N-dimethyl-3-diphenylphosphinopropionamide under identical conditions produced a liquid phase containing 164 mmole of formaldehyde remaining (42 % conversion) with 30.9 mmole glycol aldehyde (26 % selectivity) and 11.2 mmole methanol (9.2 % selectivity).

Example 9

The 125 cc stainless steel Parr bomb used in Example 2 was charged with 25.0 g sulfolane, 0.75 g paraformaldehyde (23.8 mmole), 0.082 g chlorocarbonylbis (triphenylphosphine) rhodium (I) (0.118 mmole) and 0.061 g N,N-dimethyl-3-diphenylphosphinopropionamide (0.213 mmole) which had been sparged with nitrogen. The bomb was then charged with 11.0 MPa (1 600 psi) of a 1 : 1 carbon monoxide : hydrogen gas mixture and subsequently heated to 130 °C for 3.0 hours. The bomb was then cooled to room temperature, the gases vented, and the liquid contents analyzed as 10.8 mmole formaldehyde remaining (55 % conv.), 9.1 mmole glycol aldehyde (70 % selectivity) and 5.4 mmole methanol (42 % selectivity).

Comparative Example 9

A comparative example run under identical conditions as those of example 9 except no N,N-dimethyl-3-diphenylphosphinopropionamide was used. The analysis of the liquid phase gave 3.8 mmole formaldehyde remaining (84 % conversion), 1.75 mmole glycol aldehyde (8.8 % selectivity) and 1.63 mmole methanol (8.2 % selectivity).

Example 10

The 300 cc stainless steel autoclave used in Example 1 was charged with 100 g acetonitrile, 3.0 g paraformaldehyde (95 mmole), 0.121 g dicarbonylacetylacetonato rhodium (I) (0.469 mmole), 0.054 g trifluoracetic acid (0.473 mmole), and 0.362 g N,N-dimethyl-3-diphenylphosphinopropionamide (1.27 mmole). The autoclave was then charged with 12.1 MPa (1 750 psi) carbon monoxide and 3.1 MPa (450 psi) hydrogen at room temperature and heated to 110 °C for 2 hours, whereupon the reactor was cooled and vented. The liquid contents were analyzed for 13.3 mmole formaldehyde remaining (86 % conversion), 74 mmole glycol aldehyde and 1.2 mmole ethylene glycol (92 % selectivity) and 3.4 mmole methanol (4.2 % selectivity).

Comparative Example 10

A comparative example under identical conditions using an equimolar concentration of triphenylphosphine rather than N,N-dimethyl-3-diphenylphosphinopropionamide resulted in a liquid phase containing 49.3 mmole glycol aldehyde (72 % selectivity), 3.9 mmole methanol (5.6 % selectivity) and 25.4 mmole formaldehyde remaining (73 % conversion).

Example 11

The 300 cc stainless steel autoclave equipped as in Example 1 was charged with 100 g tetraglyme, 3.0 g of 95 % paraformaldehyde (95 mmole), 0.121 g dicarbonylacetoacetonato rhodium (I) (0.469 mmole), 0.054 g trifluoracetic acid (0.470 mmole) and 0.362 g N,N-dimethyl-3-diphenylphosphinopropionamide (1.27 mmole) after a nitrogen sparge of the solution. The autoclave was sealed and flushed several times with 689 KPa (100 psi) carbon monoxide. The autoclave was then charged with 12.1 MPa (1 750 psi) of carbon monoxide and 3.1 MPa (450 psi) hydrogen at room temperature. The autoclave and its contents were heated to 110 °C and an initial pressure of 24.8 MPa (3 600 psi). A heated gas reservoir containing the same carbon monoxide : hydrogen ratio as the autoclave was used to charge the reaction autoclave when gas absorption lowered the autoclave pressure below 22.1 MPa (3 200 psi). After three fours of reaction, the autoclave was cooled and vented. Analysis indicated 24.4 mmole formaldehyde remaining (74 % conversion), 38.5 mmoles glycol aldehyde and 1.7 mmole ethylene glycol (57 % selectivity) and 20.9 mmoles methanol (30 % selectivity).

Comparative Example 11

A comparative example under identical conditions with an equimolar concentration of triphenylphosphine instead of N,N-dimethyl-3-diphenylphosphinopropionamide resulted in a liquid phase having 24.4 mmole formaldehyde remaining (74 % conversion), 22.0 mmole glycol aldehyde and 1.5 mmole ethylene glycol (33 % selectivity), and 24.1 mmole methanol (34 % selectivity).

### Example 12

The stainless steel autoclave equipped as in Example 1 was charged with 100 g acetonitrile, 3.0 g paraformaldehyde (95 mmole), 0.090 g chlorodicarbonyl rhodium (I) dimer (0.232 mmole), 0.057 g 4-dimethylaminopyridine (0.464 mmole), and 0.397 g N,N-dimethyl-3-diphenylphosphinopropionamide (1.39 mmole) which had been sparged with nitrogen. The autoclave was charged with 12.1 MPa (1 750 psi) carbon monoxide and 3.1 MPa (450 psi) hydrogen at room temperature and the autoclave and its contents were then heated to 110 °C for three hours. A gas reservoir was used to keep the pressure above 22.1 MPa (3 200 psi), as in Example 10. Analysis indicated 5.5 mmole formaldehyde remaining (94 % conversion), 64.5 mmole glycol aldehyde and 1.8 mmole ethylene glycol (74 % selectivity), and 7.1 mmole methanol (7.9 % selectivity).

### Example 13

The 300 cc stainless steel autoclave equipped as in Example 1 was charged with 100 g acetonitrile, 9.0 g paraformaldehyde (285 mmole), 0.090 g chlorodicarbonylrhodium (I) dimer (0.232 mmole), 0.114 g 4-dimethylaminopyridine (0.930 mole), and 0.397 g N,N-dimethyl-3-diphenylphosphinopropionamide. The autoclave was charged with 10.3 MPa (1 500 psi) carbon monoxide and 3.1 MPa (450 psi) hydrogen at room temperature and then heated to 100 °C for one hour. A gas reservoir having the same carbon monoxide to hydrogen ratio as the autoclave was used to keep the gas pressure above 22.1 MPa (3 200 psi). Upon completion, the autoclave was cooled and the gas vented. Analysis of the liquid solution indicated 89 mmole of formaldehyde remaining (69 % conversion), 155 mmole glycol aldehyde and 1.6 mmole ethylene glycol (80 % selectivity) and 21.5 mmole methanol (11 % selectivity).

Cobalt and ruthenium were also active for formaldehyde hydroformylation, although less active than rhodium, as is shown in the following examples.

### Example 14

The 300 cc stainless steel autoclave used in Example 1 was charged with 6.0 g paraformaldehyde (190 mmole), 100 g acetonitrile, 0.314 g ruthenium carbonyl (0.491 mmole), and 0.855 g N,N-dimethyl-3-diphenylphosphinopropionamide (3.00 mmole). The autoclave was sealed and charged with 9.7 MPa (1 400 psi) carbon monoxide and 9.3 MPa (1 350 psi) hydrogen at rom temperature and then heated to 130 °C for 2.0 hours. After cooling and venting, the analysis indicated 57.6 mmole formaldehyde remaining (70 % conversion), 6.2 mmole glycol aldehyde and 4.1 mmole ethylene glycol (7.8 % selectivity), and 5.3 mmole methanol (4.0 % selectivity).

### Example 15

The 100 cc Parr bomb equipped as in Example 2 was charged with 25 g acetonitrile, 0.75 g paraformaldehyde (23.8 mmole), 0.240 g dicobalt octacarbonyl (0.696 mmole), and 0.401 g N,N-dimethyl-3-diphenylphosphinopropionamide (1.39 mmole). The bomb was sealed and flushed twice with a 1 : 1 carbon monoxide : hydrogen mixture. The bomb was then charged with 10.3 MPa (1 500 psi) of the gas mixture, whereupon the autoclave and its-contents were heated to 130 °C for 3.0 hours, cooled to room temperature and vented. The liquid contents were analyzed as 7.21 mmoles formaldehyde remaining (69 % conversion), 2.92 mmoles glycol aldehyde (18 % selectivity) and 0.08 mmoles methanol (0.5 % selectivity).

## Claims

1. A catalyst useful for the preparation of glycol aldehyde, said catalyst being characterized by the formula :

$$MX_x(CO)_y(Z)_z$$

wherein M is a metal selected from rhodium, cobalt, ruthenium and mixtures thereof ;

X is an anion selected from halides, pseudohalides, hydrides and deprotonated strong carboxylic acids,

Z is a phosphine amide ligand having the structure

(Drawing page 12)

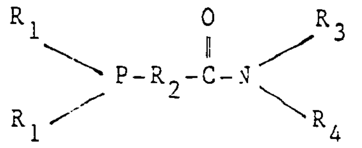

or

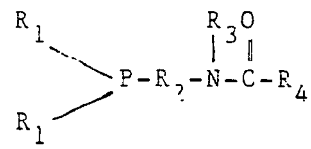

wherein

P is phosphorus

$R_1$ is an aliphatic or aromatic monovalent group containing from 1-20 carbon atoms ;

$R_2$ is a direct link or an aliphatic or aromatic divalent group containing up to 20 carbon atoms and may further comprise oxygen, nitrogen and/or sulfur atoms which may also be directly bonded to the amide C(O)N carbon or nitrogen ;

$R_3$ and $R_4$ are each aliphatic or aromatic monovalent groups containing from 1-50 carbon atoms or together form a divalent aliphatic or aromatic group

x ranges from 0 to 3, y ranges from 1 to 5, and z from 1 to 4 ;

the resultant compound being further characterized by the absence of hydrogen on the amide nitrogen atom.

2. The catalyst as claimed in claim 1 characterized in that M is rhodium.

3. The catalyst as claimed in claim 1 or claim 2 characterized in that X is an anion selected from the group of chlorides, hydrides, and trifluoroacetates.

4. The catalyst as claimed in claim 1, claim 2 or claim 3 characterized in that $R_1$ is aromatic.

5. The catalyst as claimed in any one of claims 1 to 4 characterized in that $R_2$ is aliphatic and comprises only carbon and hydrogen atoms.

6. The catalyst as claimed in any one of claims 1 to 5 characterized in that $R_3$ and $R_4$ are each aliphatic and comprise only carbon and hydrogen atoms.

7. A catalyst as claimed in any one of claims 1 to 5 wherein an additional organophosphine group is incorporated into at least one of the $R_3$ and $R_4$ groups.

8. The catalyst as claimed in claim 1 wherein the phosphine-amide ligand is N,N-dimethyl-3-diphenylphosphinopropionamide, N-methyl-N-(2-diphenylphosphinoethyl) acetamide, or N(2-diphenylphosphinoethyl)-2-pyrrolidinone.

9. A process for the preparation of glycol aldehyde comprising contacting formaldehyde, carbon monoxide and hydrogen in the presence of an effective polar or nonpolar organic solvent and catalyst under super-atmospheric pressure and elevated temperature, and separating the product glycol aldehyde from the reaction mixture, characterized in that the catalyst comprises a metal catalyst complex as claimed in any one of claims 1 to 8.

10. A process as claimed in claim 9 characterized in that carbon monoxide and hydrogen are present in a mole ratio ranging from 20 to 1 to 1 to 20 $CO/H_2$.

11. A process as claimed in claim 9 or claim 10 characterized in that the solvent is a polar solvent.

12. A process as claimed in claim 11 characterized in that the polar solvent is selected from nitriles, cyclic ethers, alkyl ethers, alkyl sulfones and sulfoxides, ketones, esters, lactones, organic acids and alkanols.

13. A process as claimed in any one of claims 9 to 12 characterized in that the superatmospheric pressure is in the range from about 140 to 280 atmospheres.

14. A process as claimed in any one of claims 9 to 13 characterized in that the elevated temperature is in the range from about 50 to 150 °C.

**Patentansprüche**

1. Katalysator, wertvoll zur Herstellung von Glykolaldehyd, gekennzeichnet durch die folgende Formel :

$$MX_x(CO)_y(Z)_z$$

in der M ein Metall, ausgewählt aus Rhodium, Kobalt, Ruthenium und Mischungen davon, bedeutet ;

X ein Anion, ausgewählt aus den Halogeniden, Pseudohalogeniden, Hydriden und deprotonierten starken Carbonsäuren bedeutet,

Z ein Phosphinamidligand mit der Struktur

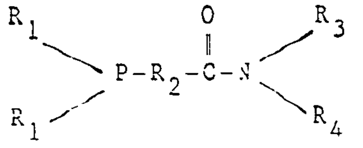

oder

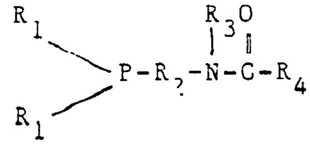

ist, worin

P Phosphor ist,

$R_1$ eine aliphatische oder aromatische monovalente Gruppe mit 1-20 Kohlenstoffatomen bedeutet ;

$R_2$ eine direkte Verbindung oder eine aliphatische oder aromatische divalente Gruppe mit bis zu 20 Kohlenstoffatomen bedeutet und zusätzlich Sauerstoff, Stickstoff und/oder Schwefelatome enthalten kann, die auch direkt an den Amid C(O)N Kohlenstoff oder Stickstoff gebunden sein können ;

$R_3$ und $R_4$ aliphatische oder aromatische monovalente Gruppen mit 1 bis 50 Kohlenstoffatomen sind oder zusammen eine divalente aliphatische oder aromatische Gruppe bilden

x 0 bis 3, y 1 bis 5 und z 1 bis 4 ist ;

und wobei die erhaltene Verbindung zusätzlich durch die Abwesenheit von Wasserstoff am Amidstickstoffatom gekennzeichnet ist.

2. Katalysator nach Anspruch 1, worin M Rhodium bedeutet.

3. Katalysator nach Anspruch 1 oder 2, worin X ein Anion, ausgewählt aus der Gruppe der Chloride, Hydride und Trifluoracetate ist.

4. Katalysator nach Anspruch 1, 2 oder 3, worin $R_1$ ein aromatischer Rest ist.

5. Katalysator nach Anspruch 1 bis 4, worin $R_2$ ein aliphatischer Rest und nur Kohlenstoff- und Wasserstoffatomen enthält.

6. Katalysator nach Anspruch 1 bis 5, worin jeder Rest $R_3$ und $R_4$ aliphatisch ist und nur Kohlenstoff- und Wasserstoffatome enthält.

7. Katalysator nach Anspruch 1 bis 5, worin eine zusätzliche Organophosphingruppe in zumindest eine der Gruppen $R_3$ und $R_4$ eingebaut ist.

8. Katalysator nach Anspruch 1, worin der Phosphinamidligand N,N-Dimethyl-3-diphenylphosphinopropionamid, N-Methyl-N-(2-diphenylphosphinoethyl) acetamid oder N(2-Diphenylphosphinoethyl)-2-pyrrolidinon, ist.

9. Verfahren zur Herstellung von Glykolaldehyd, in dem man Formaldehyd, Kohlenstoffmonoxyd und Wasserstoff in Gegenwart eines wirksamen polaren oder nicht-polaren organischen Lösungsmittels und eines Katalysators unter über-atmosphärischem Druck und erhöhter Temperatur in Kontakt bringt und das erhaltene Glykolaldehyd aus der Reaktionsmischung abtrennt, dadurch gekennzeichnet, daß der Katalysator aus einem Metallkatalysatorkomplex nach Anspruch 1 bis 8 besteht.

10. Verfahren nach Anspruch 9, worin Kohlenmonoxyd und Wasserstoff in einem Molverhältnis von 20 zu 1 bis 1 zu 20, $CO/H_2$, vorhanden sind.

11. Verfahren nach Anspruch 9 oder 10, worin das Lösungsmittel ein polares Lösungsmittel ist.

12. Verfahren nach Anspruch 11, dadurch gekennzeichnet, daß das polare Lösungsmittel ausgewählt ist aus Nitrilen, cyklischem Ether, Alkylether, Alkylsulfonen und Sulfoxyden, Ketonen, Estern, Lactonen, organischer Säure und Alkanolen.

13. Verfahren nach Anspruch 9 bis 12, worin der über-atmosphärische Druck im Bereich von etwa 140 bis 280 Atmosphären beträgt.

14. Verfahren nach Anspruch 9 bis 13, dadurch gekennzeichnet, daß die erhöhte Temperatur im Bereich von etwa 50 bis 150 °C liegt.

**Revendications**

1. Catalyseur pouvant servir à la préparation de l'aldéhyde glycolique, ledit catalyseur étant caractérisé par la formule :

$$MX_x(CO)_y(Z)_z$$

dans laquelle M représente un métal choisi parmi le rhodium, le cobalt, le ruthénium et leurs mélanges ;

X représente un anion choisi parmi des halogénures, des pseudohalogénures, des hydrures et des acides carboxyliques forts déprotonés,

Z représente un ligand phosphine-amide ayant la structure

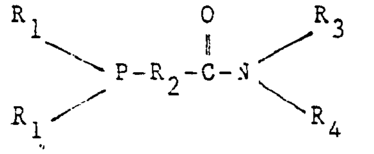

ou

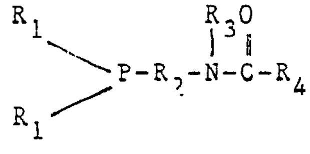

dans laquelle

P représente le phosphore,

$R_1$ est un groupe monovalent, aliphatique ou aromatique, contenant 1 à 20 atomes de carbone ;

$R_2$ est une liaison directe ou un groupe divalent, aliphatique ou aromatique, contenant jusqu'à 20 atomes de carbone et qui peut comprendre en outre des atomes d'oxygène, d'azote et/ou de soufre, pouvant aussi être directement liés au carbone ou à l'azote d'amide C(O)N ;

$R_3$ et $R_4$ représentent chacun un groupe monovalent aliphatique ou aromatique contenant 1 à 50 atomes de carbone ou bien ils forment ensemble un groupe aliphatique ou aromatique divalent,

x vaut de 0 à 3, y vaut de 1 à 5 et z vaut de 1 à 4 ;

le composé résultant étant en outre caractérisé par l'absence d'hydrogène sur l'atome d'azote de fonction amide.

2. Catalyseur selon la revendication 1, caractérisé en ce que M représente le rhodium.

3. Catalyseur selon la revendication 1 ou la revendication 2, caractérisé en que X est un anion choisi parmi les chlorures, hydrures et trifluoracétates.

4. Catalyseur selon la revendication 1, la revendication 2 ou la revendication 3, caractérisé en ce que $R_1$ est un groupe aromatique.

5. Catalyseur selon l'une quelconque des revendications 1 à 4, caractérisé en ce que $R_2$ représente un groupe aliphatique et ne comprend que des atomes de carbone et d'hydrogène.

6. Catalyseur selon l'une quelconque des revendications 1 à 5, caractérisé en ce que $R_3$ et $R_4$ représentent chacun un groupe aliphatique ne comprenant que des atomes de carbone et d'hydrogène.

7. Catalyseur selon l'une quelconque des revendications 1 à 5, dans lequel un groupe organophosphine supplémentaire est incorporé à l'un au moins des groupes $R_3$ et $R_4$.

8. Catalyseur selon la revendication 1, dans lequel le ligand phosphine-amide est le N,N-diméthyl-3-diphényl phosphinopropionamide, le N-méthyl-N-(2-diphénylphosphinoéthyl) acétamide ou la N(2-diphénylphosphinoéthyl)-2-pyrrolidinone.

9. Procédé pour la préparation de l'aldéhyde glycolique, comprenant la mise en contact du formaldéhyde, du monoxyde de carbone et de l'hydrogène en présence d'un solvant organique efficace, polaire ou non polaire, et d'un catalyseur sous une pression supérieure à la pression atmosphérique et à température élevée, et la séparation de l'aldéhyde glycolique produit d'avec le mélange réactionnel, procédé caractérisé en ce que le catalyseur comprend un complexe de catalyseur métallique selon l'une quelconque des revendications 1 à 8.

10. Procédé selon la revendication 9, caractérisé en ce que le monoxyde de carbone et l'hydrogène sont présents selon un rapport molaire compris entre 20 : 1 et 1 : 20 de $CO/H_2$.

11. Procédé selon la revendication 9 ou la revendication 10, caractérisé en ce que le solvant est un solvant polaire.

12. Procédé selon la revendication 11, caractérisé en ce que le solvant polaire est choisi parmi les nitriles, les éthers cycliques, les éthers d'alkyle, les alkyl sulfones et les sulfoxydes, les cétones, les esters, les lactones, les acides organiques et les alcanols.

13. Procédé selon l'une quelconque des revendications 9 à 12, caractérisé en ce que la pression supérieure à la pression atmosphérique se situe entre environ 140 et 280 atmosphères.

14. Procédé selon l'une quelconque des revendications 9 à 13, caractérisé en ce que la pression élevée se situe entre environ 50 et 150 °C.